# EUROPEAN PATENT SPECIFICATION

(11) **EP 1 899 474 B1**
(45) Date of publication and mention of the grant of the patent: **25.11.2009**
(21) Application number: 06754661.4
(22) Date of filing: 04.07.2006
(51) Int. Cl.: C12P 21/02, C12N 15/00

(54) **USE OF LEPA FOR IMPROVING THE ACCURACY OF PROTEIN SYNTHESIS IN VITRO**
VERWENDUNG VON LEPA ZUR VERBESSERUNG DER GENAUIGKEIT DER IN-VITRO-PROTEINSYNTHESE
UTILISATION DE LEPA POUR AMELIORER LA PRECISION DE LA SYNTHESE DES PROTEINES IN VITRO

(30) Priority: 04.07.2005 EP 05014471
(43) Date of publication of application: 19.03.2008
(73) Proprietor: Max-Planck-Gesellschaft zur Förderung der Wissenschaften e.V., 80539 München (DE)
(72) Inventor: NIERHAUS, Knud, H., 14195 Berlin (DE); QIN, Yan, 14195 Berlin (DE); WILSON, Daniel, N., 81369 München (DE)
(74) Representative: Weiss, Wolfgang
(86) International application number: PCT/EP2006/006503
(87) International publication number: WO 2007/003410

(56) References cited:
- WO-A-99/50436
- BETTON J-M: "RAPID TRANSLATION SYSTEM (RTS): A PROMISING ALTERNATIVE FOR RECOMBINANT PROTEIN PRODUCTION" CURRENT PROTEIN AND PEPTIDE SCIENCE, BENTHAM SCIENCE PULBISHERS, NL, vol. 4, no. 1, February 2003 (2003-02), pages 73-80, XP008038258 ISSN: 1389-2037
- MARTIN G A ET AL: "HIGH-YIELD, IN VITRO PROTEIN EXPRESSION USING A CONTINUOUS-EXCHANGE, COUPLED TRANSCRIPTION/TRANSLATION SYSTEM" BIOTECHNIQUES, INFORMA LIFE SCIENCES PUBLISHING, WESTBOROUGH, MA, US, vol. 31, no. 4, October 2001 (2001-10), pages 948-950,952, XP001153872 ISSN: 0736-6205
- COLCA JERRY R ET AL: "Cross-linking in the living cell locates the site of action of oxazolidinone antibiotics." JOURNAL OF BIOLOGICAL CHEMISTRY, vol. 278, no. 24, 13 June 2003 (2003-06-13), pages 21972-21979, XP002399244 ISSN: 0021-9258 cited in the application
- EHRENBERG M ET AL: "DESIGN AND USE OF A FAST AND ACCURATE IN VITRO TRANSLATION SYSTEM" PRACTICAL APPROACH SERIES, OXFORD, GB, 1990, pages 101-129, XP001022132 ISSN: 0957-025X

## Description

The present invention relates to methods, systems, compositions and kits for the synthesis of proteins *in vitro,* wherein the protein synthesis is carried out in the presence of the prokaryotic ribosomal factor LepA in order to significantly improve the accuracy of protein synthesis.

Systems for the *in vitro* synthesis of proteins are offered commercially and are important tools for structural and functional studies of proteins. Examples of the usage of these systems include the synthesis of toxic proteins that might be difficult to express in vivo, expression of heterologous proteins from organisms that might be difficult to cultivate in order to crystallize and/or to perform functional studies, synthesis of proteins doted with deuterium, ¹³C and ¹⁵N incorporation for NMR structure determination in solution, incorporation of artificial amino acids, such as selenomethionine, at specific protein positions for crystallization or pharmaceutical applications etc.

The most comprehensive and efficient *in vitro* systems for protein synthesis are coupled transcription/translation systems with bacterial cell lysates, where one adds, for example, T7 polymerase and a plasmid carrying a gene under a T7 promoter, e.g. Roche RTS 100 *E. coli* HY Kit, Roche RTS 500 *E. coli* HY Kit, Promega TNT Quick coupled Transcription/Translation Systems together with Promega *E. coli* T7 S30 Extract System for circular DNA, etc. The T7 transcript programs the translational apparatus of the lysate yielding up to 7 mg of synthesized protein per ml.

An example for such an *in vitro* system for protein synthesis is the Rapid Translation System (RTS) disclosed by Betton (2003). RTS uses a combination of a T7RNA polymerase and a bacterial extract (*E. coli* S30). The gene to be expressed is either cloned into a plasmid vector or present as a PCR product placed under the control of T7 promoter.

The major drawback of the currently available systems is the low accuracy with which the proteins are produced, i.e., the active fraction of distinct proteins can be as low as 30% of the total protein fraction for a given protein, therefore compromising the use of these protein products for subsequent molecular analysis. Surprisingly, it was found that addition of the prokaryotic ribosomal factor LepA improves the accuracy of the synthesized proteins to about 100% without significantly affecting the protein yield.
The subject-matter of the present invention is defined by the claims.

Also disclosed is a method for synthesizing a protein *in vitro* by a translation system, particularly a bacterial translation system, wherein the protein synthesis is carried out in the presence of the prokaryotic ribosomal factor LepA.

Also disclosed is an *in vitro* translation system which comprises the prokaryotic ribosomal factor LepA.

Also disclosed is a reagent composition or kit for the *in vitro* synthesis of a protein comprising the prokaryotic ribosomal factor LepA.

Still a further aspect of the present invention is the use of the prokaryotic ribosomal factor LepA for increasing the accuracy of protein synthesis.

LepA was identified as a G-protein and is one of the most conserved proteins known in biology (Genebank Swiss-Prot: LepA from *Escherichia coli*: Entry name LEPA_ECO57; Primary accession number 60787; Genebank UniProt/TrEMBL: LepA orthologue from human: Entry name Q5XKM8_HUMAN: primary accession number Q5XKM8; protein name: FLJ13220). After EF-Tu (in archaea and eukarya EF1A) LepA is the second most conserved protein known with an amino-acid identity of 48 to 85% (Caldon et al., 2001). Sequence comparison suggested that it consists of five domains, the first four of which correspond to domains 1-3 and 5 of the elongation factor EF-G, respectively. In addition, LepA has a highly conserved C-terminal domain that has no sequence homology with any known proteins.

As shown in the example of the present application, prokaryotic LepA is a ribosomal factor which is capable of improving the accuracy of a protein synthesis particularly in an *in vitro* translation system.

The translation system can be any standard cell-free translation system, e.g. a bacterial system, which is supplemented by prokaryotic LepA. The system comprises (a) a translatable RNA encoding the protein to be synthesized and (b) a cell-free preparation comprising components of the cellular translation apparatus. Perferably the system is a coupled transcription/translation system. The transcription/ translation system preferably comprises (a1) a template nucleic acid encoding the protein to be synthesized operatively linked to an expression control sequence, (a2) a polymerase capable of producing translatable RNA from the nucleic acid (a1) and (b) from which the translatable RNA can be obtained by transcription. The system is a prokaryotic system.

The system comprises translatable RNA encoding the protein to be synthesized and components of the cellular translation apparatus capable of translating the RNA. Preferably the system further comprises a template nucleic acid from which the translatable RNA can be obtained, e.g. by transcription or replication. In a preferred embodiment, the template nucleic acid is a DNA-molecule, e.g. a plasmid, encoding the protein to be synthesized operatively linked to an expression control sequence. The nucleic acid is expressed by a DNA-dependent RNA polymerase capable of transcribing the nucleic acid. On the other hand, the nucleic acid may be an RNA which may be replicated by an RNA-dependent RNA polymerase or replicase. The translatable RNA contains prokaryotic or eukaryotic translation signals, which are recognized by the components of the translation apparatus present in the system.

In a preferred embodiment, the expression control sequence is a heterologous promoter, such as a T7 or related promoter, e.g. a SP6 promoter, and the polymerase is a heterologous polymerase, such as a T7 RNA polymerase or related polymerase, e.g. SP6 RNA-polymerase.

Alternatively, the promoter may be a native cellular promoter and the polymerase is a native cellular DNA-dependent RNA polymerase.

The components of the cellular translation apparatus in the *in vitro* system are preferably provided by a translation-competent cell extract. The cell extract is preferably a cellular lysate, more preferably an extract or lysate from a prokaryotic cell, e.g. from *E.coli* cells or cells from another bacterial gram-negative prokaryotic cell or from a gram-positive prokaryotic cell, such as a *B. subtilis* cell.

In addition to the components as indicated above, the system may comprise usual components required for translation and optionally transcription or replication, such as ribonucleotides for RNA synthesis, amino acids for protein synthesis, a suitable biological energy source, such as ATP, acetylphosphate, phosphoenolpyruvate plus pyruvate kinase and similar systems.

The ribosomal factor LepA which is used to supplement the transcription/ translation system, may be a prokaryotic or eukaryotic (e.g. mitochondrial) LepA, preferably a prokaryotic LepA, e.g. a LepA protein from *E. coli*. The prokaryotic LepA protein is preferably added as a homologous component to the system. It may be added as an isolated protein, e.g. purified from native or recombinant overproducing cells, or as a partially purified cell fraction. Further, the use of functional prokaryotic LepA fragments or variants, e.g. prokaryotic LepA fragments or variants having ribosomal dependent GTPase activity still active in preventing errors are contemplated. Further contemplated are mutational altered elongation factor EF-G and fragments of EF-G that show LepA activity.

The amount of prokaryotic LepA in the system can be varied in a broad range in order to obtain a beneficial effect on the accuracy of protein synthesis without significantly reducing the efficiency of protein synthesis. For example, in a prokaryotic system, prokaryotic LepA is added in a molar ratio from about 0.05:1 to about 0.6:1, preferably from about 0.1:1 to about 0.5:1, most preferably from about 0.3:1 to about 0.4:1 to the amount of the 70S ribosomal subunit present in the system.

The methods, systems and reagent kits of the present invention are particularly suitable for the synthesis of proteins which are toxic *in vivo*, expression of proteins from organisms which are difficult to cultivate, or proteins which contain isotopes and/or artificial amino acids.

Furthermore, the present invention is to be explained in greater detail by the examples and figures hereinbelow.

### Description of Drawings

- Fig: 1: Growth curves for *E. coli* cells of the strain BL21 under various conditions shown at the right side with the same color codes as those of the curves. The arrows indicate the addition of the inducer IPTG.
- Fig. 2: Ribosome-dependent GTPase of EF-G (■) and LepA (◆). The concentration of each factor was kept constant at 0.2 µM.
- Fig. 3: Puromycin reaction of various ribosomal states. +, the peptidyl-residue of the P-tRNA is transferred to the puromycin at the A site of the peptidyl transferase center; -, no transfer occurs to puromycin.
- Fig. 4: LepA induces a back-translocation (re-TL). The blue line is the reversed DNA transcript that indicates the ribosome position before translocation (third spot), the fourth spot a position three nucleotides shorter due to a translocation reaction. The last spot shows the back-translocation after adding LepA to the post-translocational state.
- Fig. 5: A, synthesis of active GFP indicated by the fluorescent band in a native gel. B, the total synthesis derived from scanning the GFP band in an SDS gel is indicated with the blue line. The pink line indicates the amount derived from the fluorescent band of GFP in a native gel (A), the green band indicates the active fraction of the synthesized GFP. B, the active fraction of luciferase in the presence of increasing amounts of LepA, C, comparison of the LepA effect (pink, LepA:70S=0.3:1) on the active fraction of GFP (left) and luciferase (right).

### Example

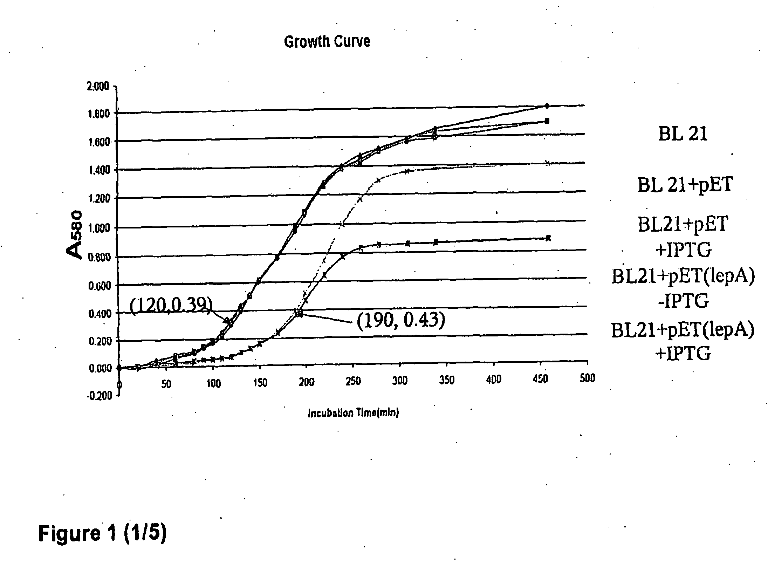

We fished the LepA gene from the *E. coli* genome and cloned it into a plasmid pET14b, which was under the control of a T7 promoter and added a His₆-tag to the N-terminus of the protein. First, we determined the effects of overexpression of LepA on the growth of *E. coli* cells BL21(DE3)physS according to manufacturers instruction (Novagen). Figure 1 shows that even without induction of the LepA expression the growth only starts after a prolonged lag phase and enters earlier into the stationary phase compared with the control strains. This is expected since in the *E. coli* expression strain the T7 polymerase is under the control of a leaky LacZ promoter, thus allowing expression of the LepA from the plasmid without IPTG induction. The growth inhibition effects were much more severe after IPTG induction of LepA expression.

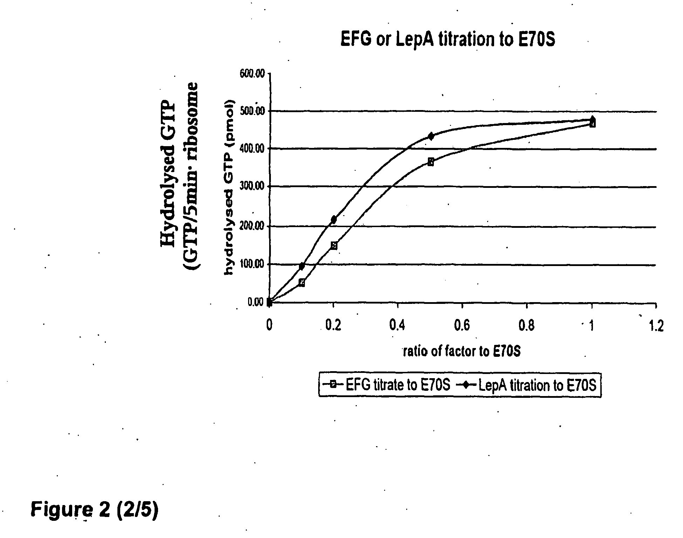

The growth stopped at a lower cell density, demonstrating that overexpression of the LepA is lethal to the cell. Next, the LepA protein was isolated after induction of expression and soluble protein purified via a Ni²⁺-column under native conditions and then tested in various functional assays. The first functional analysis was a test of a possible ribosome dependent GTPase activity of LepA according to {Dasmahapatra, 1981 #14727} with the buffer system described in {Dinos, 2004 #14684}. Since LepA might be an evolutionary offspring from the EF-G gene, we compared the LepA GTPase activity with that of EF-G, which is known to have one of the strongest ribosomal dependent GTPase activities. Figure 2 shows that LepA not only has a ribosome dependent GTPase activity but that it is at least as strong as that of EF-G. With the exception of the indirect evidence of Mankin and co-workers that LepA cross-linked to oxazolidinones only when bound to the ribosome (see Colca et al., 2003), our data provide the first strong evidence that LepA is indeed a ribosomal factor. Control experiments indicate that LepA cannot translocate the tRNA₂•mRNA complex on the ribosome as EF-G.

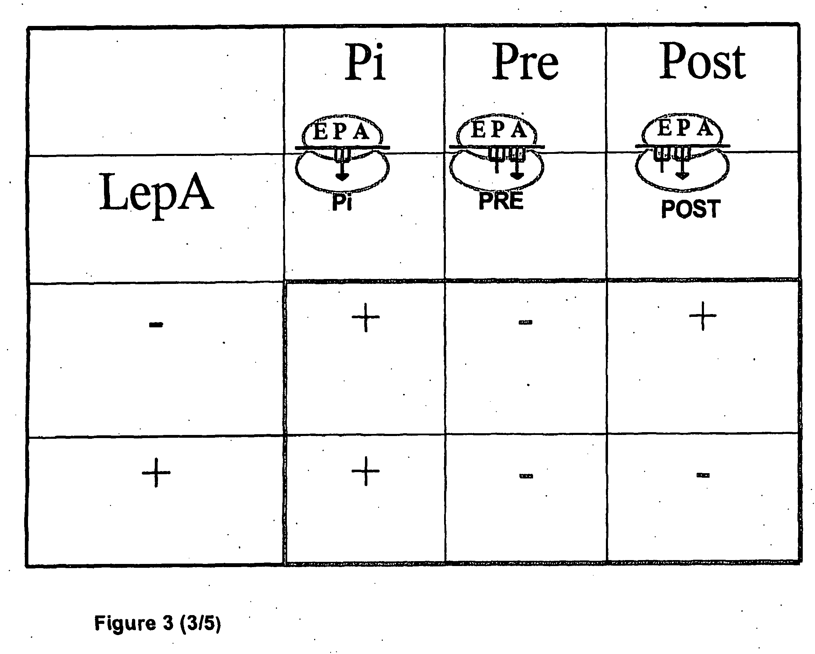

The next experiment was a surprise and gave the first hint of the function of LepA: When an analogue of a peptidyl tRNA was present at the P site and the adjacent E and A sites were free (a ribosome functional state referred to as the Pi state, i for initiation), LepA did not affect the puromycin reaction (according to {Bommer, 1996 #11801} in the buffer system described in {Dinos, 2004 #14684}), i.e. LepA did not prevent transfer of the aminoacyl moiety of the P site tRNA to the antibiotic puromycin that binds in the ribosomal A site of the peptidyltransferase centre. In contrast, in the post-translocational state LepA prevented a puromycin reaction (Figure 3).

With the more laborious dipeptide analysis (see for example Marquez et al., 2004), this finding could be confirmed. The only possible explanation for these results was that LepA induces a so-called "back-translocation" whereby the tRNAs are moved back from the P and E sites to the A and P sites and thus the puromycin reaction is prevented because the A site tRNA occupied the binding site of puromycin.

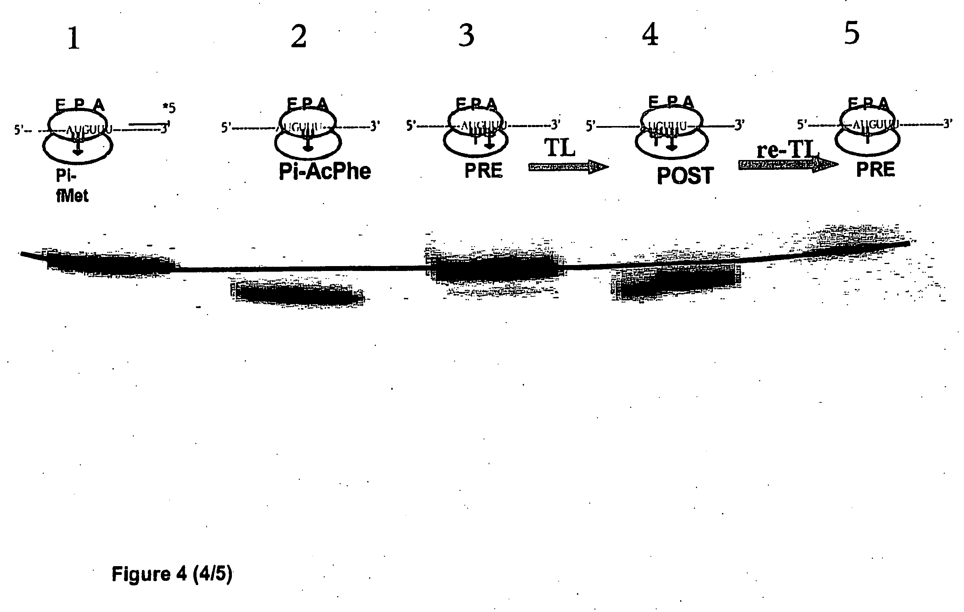

A direct test of this interpretation is a determination of the effect of LepA on the position of the mRNA relative to the ribosome using various ribosomal functional states. This method is called a "footprinting assay" using reversed transcription as described in {Connell, 2002 #13483}. The assay measures the distance (via reverse-transcription) from a fixed point in the mRNA downstream of the ribosome (determined by a DNA primer complementary to a mRNA) to the ribosome. If the ribosome makes a translocation, the distance becomes shorter since the mRNA moves into the ribosome (in the 5' direction), whereas if the ribosome makes a back-translocation the distance is increased. This is illustrated in Figure 4. When LepA is added to a ribosome in a post-translocational state, the distance becomes longer (E). Therefore, LepA obviously has a unique function compared with all other known translocational factors since it induces a back-translocation.

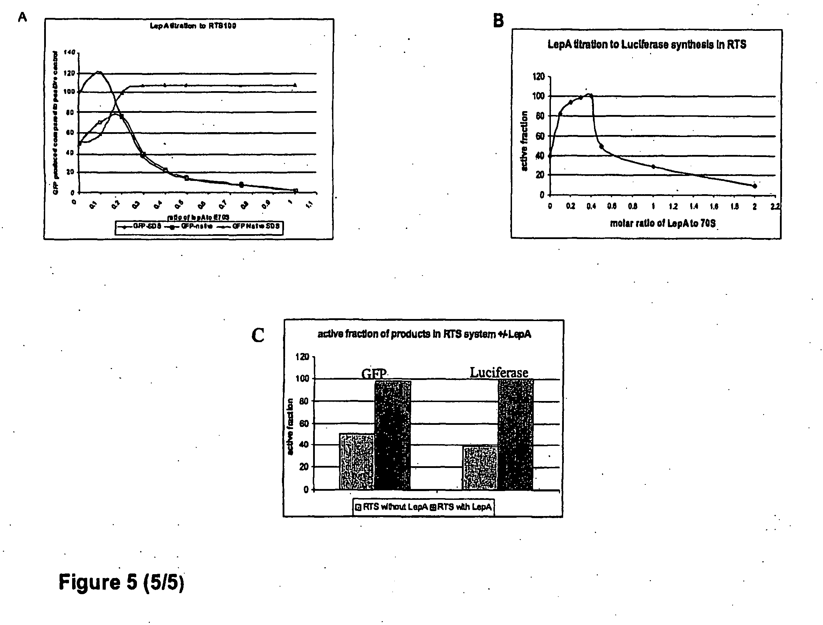

What is the function of this surprising back-translocation activity? Figure 5B provides a possible answer. It shows the effect of LepA on the synthesis of GFP in a coupled transcription/translation system. At 0 of the x-axis, the 100% value (blue line) indicates the total synthesis derived from the GFP band intensity of an SDS gel electrophoresis. The pink spot again at the 0 position of the x-axis shows the active amount of synthesized GFP determined with a native gel electrophoresis shown in Figure 5A (the active fraction was determined according to {Dinos, 2004 #14684}). In this experiment the active fraction of the synthesized GFP is about 50% in the absence of LepA (green line). Addition of LepA in a molar ratio to 70S of 0.2:1 shows a small reduction of the total yield for about 20%, but an increase of the active fraction to -100%. Further additions of LepA inhibit protein synthesis proportionally, eventually blocking it completely (>1 LepA per ribosome).

These *in vitro* results are consistent with the lethal effect observed *in vivo* (as shown in Figure 1). What is particularly interesting/important is that all points during the decline of protein synthesis, the synthesized GFP is 100% active (green line in Figure 5B).

### Summary and Conclusion

The results demonstrate that (i) the G-protein LepA is a ribosomal factor with a ribosome dependent GTPase that is at least as strong as EF-G. In spite of the structural relationship to EF-G it cannot translocate the tRNA₂•mRNA complex. In fact, LepA induces the reverse reaction, namely a back-translocation that is probably related to the lack of the EF-G domain IV that might act as a "door-stop" to prevent back-translocation. LepA heals the most important drawback of the current coupled translation systems, namely the inaccuracy of the current systems: The inactive fraction can be as large as 70% of the totally synthesized protein.

Addition of suitable amounts of LepA slightly reduces the total synthesis but increases the active fraction to virtually 100%. This is important if the structures of synthesized proteins should be determined *via* crystallization or after doting the synthesized protein with isotopes such as ¹³C or ¹⁵N for NMR. Likewise, an analysis of the function of the synthesized protein becomes prohibitively difficult by a large inactive fraction of the protein under observation. These drawbacks are overcome by the present invention.

### References

Andersen, G.R., Nissen, P. And Nyborg, J. 2003. Elongation factors in protein biosynthesis. Trends Biochem. Sci. 28:434-441.
Butland, G., Peregrin-Alvarez, J.M., Li, J., Yang, W., Yang X., Canadien, V. Starostine, A., Richards, D., Beattie, B., Krogan, N., et al. 2005. Interaction network containing conserved and essential protein complexes in Escherichia coli. Nature 433: 531-537.
Caldon, C.E., Yoong, P. and March, P.E. 2001. Evolution of a molecular switch: universal bacterial GTPases regulate ribosome function. Mol. Microbiol. 41: 289-297.
Colca, J.R., McDonald, W.G., Waldon, D.J., Thomasco, L.M., Gadwood, R.C., Lund, E.T., Cavey, G.S., Mathews, W.R., Adams, L.D., Cecil; E.T. et al. 2003. Crosslinking in the living cell locates the site of action of oxazolidinone antibiotics. J. Biol. Chem. 278:21972-21979.
Marquez, V., Wilson, D.N., Tate, W.P., Triana-Alonso, F. and Nierhaus, K.H. 2004. Maintaining the ribosomal reading frame: The influence of the E site during translational regulation of release factor 2. Cell 118: 45-55.
Nierhaus, K.H. 1996. Protein synthesis - An elongation factor turn-on. Nature 379: 491-492.

## Claims

1. A method for synthesizing a protein in vitro by a prokaryotic translation system, wherein protein synthesis is carried out in the presence of the prokaryotic ribosomal factor LepA, said prokaryotic ribosomal factor LepA being added as an isolated protein or as a partially purified cell fraction in a molar ratio from 0.05:1 to 0.6:1 to the 70S ribosomal subunit present in the system.

2. The method according to claim 1, wherein the translation system comprises (a) a translatable RNA encoding the protein; and (b) a cell-free preparation comprising components of the cellular translation apparatus.

3. The method according to any one of claims 1-2, wherein the cell-free preparation is a cell extract.

4. The method according to any one of claims 1-2, wherein the cell-free preparation is a cell lysate.

5. The method according to claim 3, wherein the cell extract is an extract from a prokaryotic cell.

6. The method according to claim 5, wherein the cell extract is an extract from an *E.coli* cell.

7. The method according to claims 1-2, wherein the system is a coupled transcription/translation system.

8. The method according to claim 7, wherein the transcription/translation system comprises (a1) a nucleic acid encoding the protein to be synthesized operatively linked to an expression control sequence; (a2) a polymerase capable of producing translatable RNA from the nucleic acid and (b) a cell-free preparation comprising components of the cellular translation apparatus.

9. The method according to claim 8, wherein the expression control sequence is a heterologous promoter, such as a T7 or related promoter, and the polymerase is a heterologous polymerase, such as a T7 RNA polymerase or a related RNA polymerase, or wherein the expression control sequence is a native cellular promoter and the polymerase is a native cellular DNA-dependent polymerase.

10. The method according to any one of claims 1-9, wherein the LepA is from *E. coli.*

11. A prokaryotic *in vitro* translation system which comprises added prokaryotic ribosomal factor LepA as an isolated protein or as a partially purified cell fraction in a molar ratio from 0.05:1 to 0.6:1 to the 70S ribosomal subunit present in the system.

12. The system of claim 11 comprising (a) a translatable RNA encoding the protein to be synthesized operatively linked to an expression control sequence; (b) a cell-free preparation comprising components of the cellular translation apparatus, and (c) added ribosomal factor LepA.

13. The system of claim 11 or 12, which is a coupled transcription/ translation system.

14. A reagent composition or kit for the *in vitro* synthesis of a protein comprising added prokaryotic ribosomal factor LepA, as an isolated protein or as a partially purified cell fraction in a molar ratio from 0.05:1 to 0.6:1 to the 70S ribosomal subunit present in the composition or kit.

15. Use of the prokaryotic ribosomal factor LepA for increasing the accuracy of protein synthesis in an *in vitro* prokaryotic system.

16. The use of claim 15 in an *in vitro* coupled transcription/translation system.

## Patentansprüche

1. Verfahren für die Synthese eines Proteins *in vitro* mittels eines prokaryotischen Translationssystems, wobei die Proteinsynthese in Gegenwart des prokaryotischen ribosomalen Faktors LepA durchgeführt wird, wobei der prokaryotische ribosomale Faktor LepA als isoliertes Protein oder als teilweise gereinigte Zellfraktion in einem Molverhältnis von 0,05:1 bis 0,6:1 zu der in dem System vorhandenen ribosomalen Untereinheit zugesetzt wird.

2. Verfahren nach Anspruch 1, wobei das Translationssystem (a) eine translatierbare RNA, die für das Protein kodiert; und (b) eine zellfreie Präparation, umfassend Bestandteile des zellulären Translationsapparates, umfasst.

3. Verfahren nach einem der Ansprüche 1-2, wobei die zellfreie Präparation ein Zellextrakt ist.

4. Verfahren nach einem der Ansprüche 1-2, wobei die zellfreie Präparation ein Zelllysat ist.

5. Verfahren nach Anspruch 3, wobei der Zellextrakt ein Extrakt aus einer prokaryotischen Zelle ist.

6. Verfahren nach Anspruch 5, wobei der Zellextrakt ein Extrakt aus einer *E. coli*-Zelle ist.

7. Verfahren nach den Ansprüchen 1-2, wobei das System ein gekoppeltes Transkriptions-/Translationssystem ist.

8. Verfahren nach Anspruch 7, wobei das Transkriptions-/Translationssystem (a1) eine Nukleinsäure, die für das zu synthetisierende Protein kodiert, die operativ mit einer Expressionskontrollsequenz verbunden ist; (a2) eine Polymerase, die dazu fähig ist, translatierbare RNA von der Nukleinsäure herzustellen und (b) eine zellfreie Präparation, umfassend Bestandteile des zellulären Translationsapparates, umfasst.

9. Verfahren nach Anspruch 8, wobei die Expressionskontrollsequenz ein heterologer Promotor, wie z.B. ein T7- oder verwandter Promotor ist, und die Polymerase eine heterologe Polymerase, wie z.B. eine T7-RNA-Polymerase oder eine verwandte RNA-Polymerase ist, oder wobei die Expressionskontrollsequenz ein nativer zellulärer Promotor ist, und die Polymerase eine native zelluläre DNA-abhängige Polymerase ist.

10. Verfahren nach einem der Ansprüche 1-9, wobei das LepA aus *E. coli* ist.

11. Prokaryotisches *in vitro*-Translationssystem, das zugesetzten prokaryotischen ribosomalen Faktor LepA als isoliertes Protein oder als teilweise gereinigte Zellfraktion in einem Molverhältnis von 0,05:1 bis 0,6:1 zu der in dem System vorhandenen ribosomalen 70S-Untereinheit umfasst.

12. System nach Anspruch 11, umfassend (a) eine translatierbare RNA, die für das zu synthetisierende Protein kodiert, die operativ mit einer Expressionskontrollsequenz verbunden ist; (b) eine zellfreie Präparation, umfassend Bestandteile des zellulären Translationsapparates, und (c) zugesetzten ribosomalen Faktor LepA.

13. System nach Anspruch 11 oder 12, das ein gekoppeltes Transkriptions-/ Translationssystem ist.

14. Reagenzienzusammensetzung oder Kit für die *in vitro*-Synthese eines Proteins, die zugesetzten prokaryotischen ribosomalen Faktor LepA, als isoliertes Protein oder als teilweise gereinigte Zellfraktion in einem Molverhältnis von 0,05:1 bis 0,6:1 zu der in der Zusammensetzung oder dem Kit vorhandenen ribosomalen 70S-Untereinheit umfasst.

15. Verwendung des prokaryotischen ribosomalen Faktors LepA zur Erhöhung der Genauigkeit der Proteinsynthese in einem prokaryotischen *in vitro*-System.

16. Verwendung nach Anspruch 15 in einem gekoppelten *in vitro-*Transkriptions/Translationssystem.

## Revendications

1. Procédé de synthèse d'une protéine *in vitro* par un système de traduction procaryote, dans lequel la synthèse protéique est effectuée en présence du facteur ribosomal procaryote LepA, ledit facteur ribosomal procaryote LepA étant ajouté en tant que protéine isolée ou en tant que fraction cellulaire partiellement purifiée dans un rapport molaire de 0,05/1 à 0,6/1 par rapport à la sous-unité ribosomique 70S présente dans le système.

2. Procédé selon la revendication 1, dans lequel le système de traduction comprend (a) un ARN traduisible codant pour la protéine ; et (b) une préparation acellulaire comprenant des composants de l'appareil de traduction cellulaire.

3. Procédé selon l'une quelconque des revendications 1 et 2, dans lequel la préparation acellulaire est un extrait cellulaire.

4. Procédé selon l'une quelconque des revendications 1 et 2, dans lequel la préparation acellulaire est un lysat cellulaire.

5. Procédé selon la revendication 3, dans lequel l'extrait cellulaire est un extrait d'une cellule procaryote.

6. Procédé selon la revendication 5, dans lequel l'extrait cellulaire est un extrait d'une cellule d'*E. coli.*

7. Procédé selon les revendications 1 et 2, dans lequel le système est un système couplé de transcription/traduction.

8. Procédé selon la revendication 7, dans lequel le système de transcription/traduction comprend (a1) un acide nucléique codant pour la protéine à synthétiser, lié de manière opérationnelle à une séquence de contrôle de l'expression ; (a2) une polymérase capable de produire un ARN traduisible à partir de l'acide nucléique et (b) une préparation acellulaire comprenant des composants de l'appareil de traduction cellulaire.

9. Procédé selon la revendication 8, dans lequel la séquence de contrôle de l'expression est un promoteur hétérologue, tel qu'un promoteur T7 ou un promoteur associé, et la polymérase est une polymérase hétérologue, telle qu'une ARN polymérase T7 ou une ARN polymérase associée, ou dans lequel la séquence de contrôle de l'expression est un promoteur cellulaire natif et la polymérase est une polymérise dépendant de l'ADN cellulaire natif.

10. Procédé selon l'une quelconque des revendications 1 à 9, dans lequel le facteur LepA provient d'*E. coli.*

11. Système de traduction procaryote *in vitro* qui comprend le facteur ribosomal procaryote LepA ajouté en tant que protéine isolée ou en tant que fraction cellulaire partiellement purifiée dans un rapport molaire de 0,05/1 à 0,6/1 par rapport à la sous-unité ribosomique 70S présente dans le système.

12. Système selon la revendication 11, comprenant (a) un ARN traduisible codant pour la protéine à synthétiser, lié de manière opérationnelle à une séquence de contrôle de l'expression ; (b) une préparation acellulaire comprenant des composants de l'appareil de traduction cellulaire, et (c) le facteur ribosomal LepA ajouté.

13. Système selon la revendication 11 ou 12, qui est un système couplé de transcription/traduction.

14. Composition ou kit de réactif pour la synthèse *in vitro* d'une protéine comprenant le facteur ribosomal procaryote LepA ajouté, en tant que protéine isolée ou en tant que fraction cellulaire partiellement purifiée dans un rapport molaire de 0,05/1 à 0,6/1 par rapport à la sous-unité ribosomique 70S présente dans la composition ou le kit.

15. Utilisation du facteur ribosomal procaryote LepA pour accroître la précision de la synthèse des protéines dans un système procaryote *in vitro.*

16. Utilisation selon la revendication 15 dans un système couplé de transcription/traduction *in vitro.*
